# EUROPÄISCHE PATENTSCHRIFT

(11) **EP 2 457 058 B1**
(45) Veröffentlichungstag und Bekanntmachung des Hinweises auf die Patenterteilung: **02.09.2015**
(21) Anmeldenummer: 10734084.6
(22) Anmeldetag: 08.07.2010
(51) Int. Cl.: G01B 11/24, A61B 1/04, A61C 19/04

(54) **GENERIERUNG EINES GESAMTDATENSATZES**
GENERATING A TOTAL DATA SET
PRODUCTION D'UN ENSEMBLE DE DONNÉES GLOBALES

(30) Priorität: 24.07.2009 DE 102009026248
(43) Veröffentlichungstag der Anmeldung: 30.05.2012
(73) Patentinhaber: DeguDent GmbH, 63457 Hanau (DE)
(72) Erfinder: ERTL, Thomas, 63691 Ranstadt (DE)
(74) Vertreter: Stoffregen, Hans-Herbert
(86) Internationale Anmeldenummer: PCT/EP2010/059819
(87) Internationale Veröffentlichungsnummer: WO 2011/009736

(56) Entgegenhaltungen:
- DE-A1- 10 063 293
- US-A1- 2006 093 206
- US-A1- 2006 228 010
- US-A1- 2007 276 184
- US-A1- 2009 004 948

## Beschreibung

Die Erfindung bezieht sich auf die Generierung eines Gesamtdatensatzes von zumindest einem Abschnitt eines Objekts, wie Kieferbereich, zur Ermittlung zumindest eines Charakteristikums, wie Form und Lage, durch Zusammenfügung von Einzeldatensätzen, die mittels eines relativ zu dem Objekt sich bewegenden optischen Sensors als ersten Sensor und einer Bildverarbeitung ermittelt werden, wobei Einzeldatensätze von aufeinanderfolgenden Aufnahmen des Objekts redundante Daten enthalten, die zum Zusammenfügen der einzelnen Datensätze gematcht werden.

Durch intraorales Scannen eines Kieferbereichs können 3D-Daten generiert werden, auf deren Basis im CAD/CAM-Verfahren ein Zahnersatz hergestellt werden kann. Allerdings ist beim intraoralen Scannen von Zähnen der sichtbare Teilbereich eines Zahns oder Kieferabschnitts, von dem die 3D-Daten gemessen werden, zumeist viel kleiner als der gesamte Zahn oder Kiefer, so dass die Notwendigkeit besteht, mehrere Bilder bzw. die von diesen abgeleiteten Daten zu einem Gesamtdatensatz des Zahns oder Kieferabschnitts zu vereinigen.

Üblicherweise wird ein optischer Sensor wie 3D-Kamera von Hand geführt, um kontinuierlich die relevanten Bereiche eines Kiefers zu erfassen, um sodann mittels einer Bildverarbeitung aus den einzelnen Bildern 3D-Daten zu generieren, aus denen anschließend ein Gesamtdatensatz erstellt wird. Da die Bewegung von Hand erfolgt, kann nicht sichergestellt werden, dass bei schneller Sensorbewegung hinreichend genug Daten zur Verfügung stehen. Bei zu langsamer Bewegung ergeben sich zu viele redundante Daten in bestimmten Bereichen des Objekts. Redundante Daten sind dabei diejenigen, die sich aus Überlappungen von aufeinanderfolgenden Bildern ergeben, d.h., dass die redundanten Daten diejenigen sind, die aus dem Überlappungsbereich erzeugt werden.

Um diese Risiken auszuschließen, ist eine hohe konstante Bildwiederholrate erforderlich, um auch im Fall einer schnellen Bewegung genügend Daten mit ausreichendem Überlappungsgrad der einzelnen Datensätze zu erhalten. Eine aufwendige Elektronik mit hoher Bandbreite und ein großer Speicherbedarf sind die Folgen hiervon.

Aus der US-A-2006/0093206 ist ein Verfahren zur Ermittlung eines 3D-Datensatzes aus 2D-Punktwolken zu entnehmen. Hierzu wird ein Objekt wie Zahn gescannt, wobei die Frame-Rate von der Geschwindigkeit des Scanners abhängig ist, mit dem die Aufnahmen vorgenommen werden.

Die US-A-2006/0212260 bezieht sich auf ein Verfahren zum Scannen eines intraoralen Hohlraums. Dabei wird der Abstand zwischen einer scannenden Vorrichtung und einem zu messenden Bereich zur Auswertung der Datensätze berücksichtigt.

Gegenstand der US-B-6,542,249 sind ein Verfahren und eine Vorrichtung zum dreidimensionalen berührungslosen Scannen von Gegenständen. Einzelaufnahmen überlappen sich, um 3D-Daten einer Oberfläche zu erhalten.

Ein gattungsbildendes Verfahren ist der US-A-2007/0276184 zu entnehmen. Dabei wird ein Endoskop in eine Körperöffnung eingeführt. Um die Bewegung des Endoskops zu ermitteln, ist ein stationärer Sensor vorgesehen, der Markierungen auf dem Endoskop erfasst.

Um einen Kieferbereich 3-dimensional zu erfassen, wird nach der US-A-2006/0228010 ein Scanner benutzt, dessen Framerate in Abhängigkeit von einer vorgegebenen Blitzlichtrate gesteuert wird, mit der der Kieferbereich beleuchtet wird.

Um nach der US-A-2009/0004948 mit einem Fahrzeug eines Spielzeugsystems rüttelfreie Bilder aufzunehmen, sind entlang des Fahrwegs Markierungen angeordnet, über die die Geschwindigkeit ermittelt wird. In Abhängigkeit von dieser wird die Bildwiederholungsrate verändert.

Der vorliegenden Erfindung liegt die Aufgabe zugrunde, ein Verfahren der eingangs genannten Art so weiterzubilden, dass die beim Scannen des Objekts gewonnenen Daten in ihrer Menge hinreichend sind, dass eine optimale Auswertung erfolgen kann, ohne dass jedoch eine zu hohe Datenmenge zu verarbeiten ist, die zu einer aufwendigen Elektronik mit hoher Bandbreite und großem Speicherbedarf führen würde.

Zur Lösung der Aufgabe sieht die Erfindung im Wesentlichen vor, dass als optischer Sensor eine 3D-Kamera verwendet wird, und dass entweder in Abhängigkeit von der Relativbewegung zwischen dem optischen Sensor und dem Objekt die Anzahl der pro Zeiteinheit ermittelten Einzeldatensätze variiert wird, wobei zur Ermittlung der Relativbewegung der erste Sensor einen zweiten Sensor aus der Gruppe Beschleunigungssensor, Rotationssensor, Intertialplattform aufweist, oder in Abhängigkeit von der Anzahl der redundanten Daten von aufeinanderfolgenden Datensätzen die Anzahl der pro Zeiteinheit ermittelten Einzeldatensätze geregelt wird. Erfindungsgemäß ist vorgesehen, dass die Datenerfassungsrate in Abhängigkeit von der Relativbewegung des optischen Sensors zum Objekt variiert wird. Es werden die einzelnen Datensätze diskontinuierlich ermittelt. Dies bedeutet, dass die Bildwiederholungsrate während des Scannvorgangs nicht konstant ist, sondern parameterabhängig. Parameterabhängig bedeutet dabei, dass z.B. relative Geschwindigkeit zwischen dem Objekt und dem optischen Sensor und/oder Abstand zwischen Sensor und zu messendem Objekt und/oder Überlappungsgrad von zwei aufeinanderfolgenden Bildern berücksichtigt wird.

Insbesondere ist vorgesehen, dass in Abhängigkeit von der Anzahl der redundanten Daten von aufeinanderfolgenden Datensätzen die Anzahl der ermittelten Einzeldatensätze pro Zeiteinheit geregelt wird. Es besteht jedoch auch die Möglichkeit, dass in Abhängigkeit der Relativgeschwindigkeit zwischen dem Objekt und dem optischen Sensor die Anzahl der zu ermittelnden Einzeldatensätze gesteuert wird.

Von der Erfindung ist jedoch auch nicht der Gedanke ausgeschlossen, dass nach einer Erfassung mit permanent hoher Datenrate redundante Bilder mit hohem Überlappungsgrad im Registrierprozess weggelassen werden. Hierdurch wird jedoch das Problem der hohen Bandbreite während der Datenerfassung nicht vollständig gelöst.

Daher ist erfindungsgemäß insbesondere vorgesehen, dass eine nachlaufende Änderung der Datenerfassungsrate unterbleibt, wie dies bei einer Steuerung über den aktuellen Überlappungsgrad bei einem Realtime-Registrierungsprozess der Fall wäre, da erst aus zwei oder mehreren aufeinanderfolgenden Datensätzen der Überlappungsgrad berechnet werden kann.

Da eine Abhängigkeit der Anzahl der Einzeldatensätze pro Zeiteinheit von der Relativbewegung zwischen dem optischen Sensor und dem Objekt abhängig ist, wird neben der Bewegung des Sensors zusätzlich die Bewegung des Objekts berücksichtigt. Die Bewegung des Objekts kann dabei mittels einer Inertialplattform oder eines geeigneten Beschleunigungssensors ermittelt werden. Durch diese Maßnahme kann die Relativbewegung zwischen dem Sensor und dem Objekt als auch die Bewegung des Objekts selbst ermittelt und bei Bedarf die Datenerfassungsrate angepasst werden.

In Weiterbildung der Erfindung ist vorgesehen, dass die Anzahl der zu ermittelnden Einzeldatensätze insbesondere bei durch Drehbewegung erfolgter Relativbewegung in Abhängigkeit vom Abstand zwischen dem optischen Sensor und zu messendem Objekt bzw. Abschnitt des Objekts verändert wird.

Das Verfahren wird mittels einer 3D-Kamera mit einem Chip wie CCD-Chip durchgeführt, der ausgelesen und sodann die Daten mittels einer Bildverarbeitung ausgewertet werden. Dabei wird der Chip in Abhängigkeit von der Relativbewegung zwischen dem optischen Sensor und dem Objekt ausgelesen. Insbesondere wird die Framerate des Chips in Abhängigkeit von Relativgeschwindigkeit zwischen dem Sensor und dem Objekt gesteuert. Es besteht jedoch auch die Möglichkeit, die Framerate des Chips in Abhängigkeit vom Überlappungsbereich von mit dem Chip aufgenommenen und aufeinanderfolgenden Bildern geregelt wird.

Der Abstand zwischen dem optischen Sensor und dem zu messenden Objekt sollte zwischen 2 mm und 20 mm liegen. Des Weiteren sollte eine Beabstandung derart erfolgen, dass das Messfeld 10 mm x 10 mm beträgt.

Aufgrund der erfindungsgemäßen Lehre wird aus der aktuellen Bewegung des optischen Sensors wie der 3D-Kamera die Datenerfassungsrate diskontinuierlich optimal festgelegt, um beste Registrierergebnisse, also Match-Ergebnisse, bei minimalem Bedarf an Speicher und Bandbreite zu erreichen.

Die Einzeldatensätze werden mit Hilfe einer geeigneten Software gematcht, also registriert, um sodann einen Gesamtdatensatz zu generieren, der bei einer zahntechnischen Anwendung Form und Lage eines Kieferbereichs repräsentiert, der mit einem Zahnersatz versehen werden soll, und auf dessen Basis der Zahnersatz im z.B. CAD/CAM-Verfahren herstellbar ist.

Als besonders wichtig und vorteilhaft ist die Kontrolle der Rotation, d.h. der Drehbewegung zur Längsachse des optischen Sensors wie Erfassungskamera zu bezeichnen, da relativ schnell hohe Rotationsgeschwindigkeiten erreicht werden. In einem kostenoptimierten System sollte die Erfassung dieser Achse allen anderen vorgezogen werden.

Bei einer Rotationseifassung ist zudem die Erfassung des Abstands zwischen zu erfassendem Objekt und dem optischen Sensor wie 3D-Kamera sinnvoll, da die erreichbaren Überlappungsgrade auch vom Abstand abhängen.

Dies erfolgt durch Auswertung einer Histogrammfunktion über die Abstände zwischen Kamera und aller oder auch nur einiger weniger Einzelmesspunkte des zu messenden Objekts.

Sodann kann der Objektabstand als Mittelwert der gültigen Messpunkte angenommen werden. In Verbindung mit der aktuellen Drehrate kann sodann die notwendige Datenerfassungsrate eingestellt werden.

Nachstehend soll anhand von Tabellen verdeutlicht werden, wie die Datenerfassungsrate (Hz) in Abhängigkeit von der Translations- bzw. Rotationsgeschwindigkeit und des erforderlichen Überlappungsgrads variiert werden kann, wobei von einem Messfeld von 10 mm x 10 mm ausgegangen wird.

Tabelle 1 zeigt für Translationsbewegungen die Datenerfassungsrate (Hz) in Abhängigkeit von der Translationsgeschwindigkeit und des erforderlichen Überlappungsgrades.

**Tabelle 1**

| | Überlappungsgrad | | | |
|---|---|---|---|---|
| Translationsgeschwindigkeit [mm/s] | 80% | 90% | 95% | 99% |
| 0,5 | 0,25 | 0,5 | 1 | 5 |
| 1 | 0,5 | 1 | 2 | 10 |
| 5 | 2,5 | 5 | 10 | 50 |
| 10 | 5 | 10 | 20 | 100 |
| 50 | 25 | 50 | 100 | 500 |

Tabelle 2 zeigt für Rotationsbewegungen die Datenerfassungsrate (Hz) in Abhängigkeit von der Rotationsgeschwindigkeit, des Abstands zum Objekt und des erforderlichen Überlappungsgrades.

**Tabelle 2**

| Abstand zum Objekt 20 mm | Überlappungsgrad | | | |
|---|---|---|---|---|
| Rotationsgeschwindigkeit [°/s] | 80% | 90% | 95% | 99% |
| 2 | 0,35 | 0,7 | 1,4 | 7 |
| 10 | 1,7 | 3,5 | 7 | 35 |
| 30 | 5,2 | 10,4 | 21 | 104 |
| 60 | 10,4 | 21 | 42 | 210 |
| 90 | 15 | 31 | 62 | 310 |

Aus den Tabellen wird ersichtlich, dass dann, wenn z.B. ein Überlappungsgrad von 90 % von zwei aufeinanderfolgenden Bildern für erforderlich erachtet wird, um das Objekt im hinreichenden Umfang messen zu können, pro Sekunde ein Bild aufzunehmen ist, sofern die Translationsgeschwindigkeit 1 mm/sec beträgt. Bei höheren Geschwindigkeit wie z.B. 50 mm/sec und einem Überlappungsgrad von 99 % müsste die Bildwiederholungsrate 500/sec betragen.

Die Tabelle 2 verdeutlicht, dass z.B. bei einer Rotationsgeschwindigkeit von 30 °/sec und einem Überlappungsgrad von 95 % 21 Aufnahmen/sec erfolgen müssen.

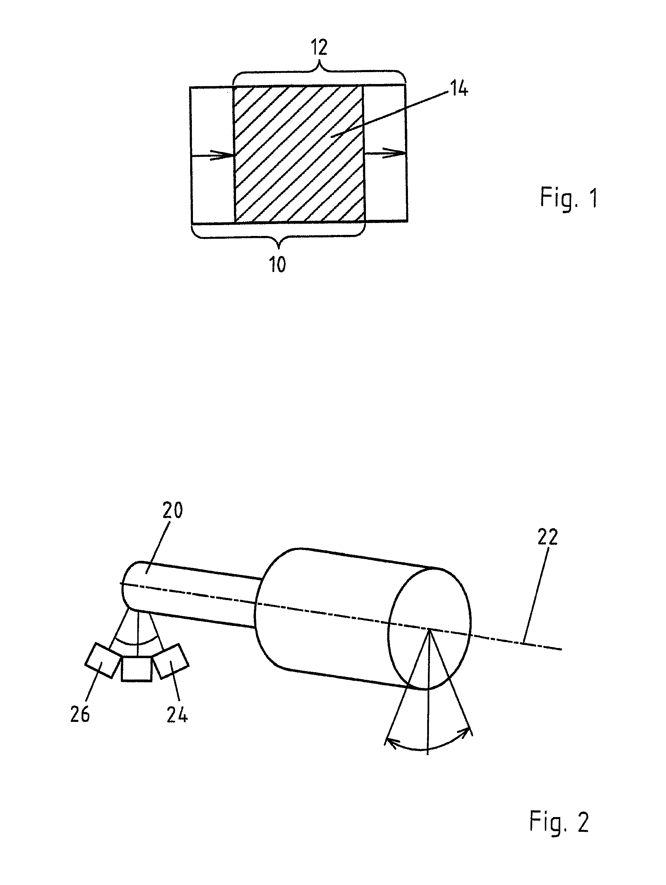

Die Überlappung zweier Bilder bei einer Translationsbewegung ist aus Fig. 1 ersichtlich. Man erkennt ein erstes Messfeld 10 und ein dieses überlappendes folgendes zweites Messfeld 12, wobei der Überlappungsbereich mit 14 gekennzeichnet ist. Die Überlappung erfolgt aufgrund einer translatorischen Bewegung und des optischen Sensors. Bei den Bildern handelt es sich um zwei sequenzielle, also unmittelbar nacheinander aufgenommene Messfelder.

In Abhängigkeit von dem Überlappungsgrad und den diesem entsprechenden Daten, die mittels eines Bildverarbeitungssystems aus den Bild- wie Grauwerten gewonnen werden, ist sodann die Bildaufnahme und damit Datenerfassungsrate zu variieren. Je geringer der Überlappungsbereich gewählt wird, umso geringer ist die Datenerfassungsrate einzustellen. Dabei kann entsprechend der erfindungsgemäßen Lehre die Datenerfassungsrate in Abhängigkeit von der Translationsgeschwindigkeit gesteuert werden.

Die Fig. 2 verdeutlicht prinzipiell, dass durch Rotation eines optischen Sensors 20 um dessen Längsachse 22 sich das jeweilige Messfeld 24, 26, also der Bild- und damit der Datenerfassungsbereich in Abhängigkeit von der Drehung um die Längsachse 22 verändert. Zum Messen eines Objekts ist es gleichfalls erforderlich, dass die Bildwiederholungsrate, also die Framerate bei einem Chip in Abhängigkeit von dem Überlappungsbereich variiert wird, wobei der Grad der Überlappung von der Rotationsgeschwindigkeit abhängig ist. Je höher die Rotationsgeschwindigkeit, umso höher muss die Bildwiederholungsrate sein, sofern der Überlappungsbereich konstant sein soll.

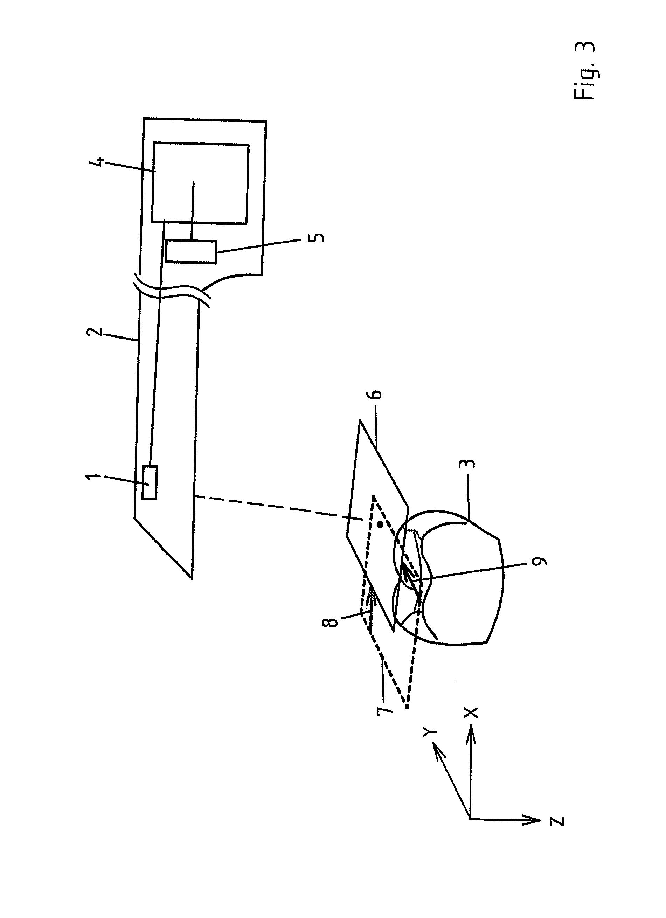

Der Fig. 3 ist noch einmal das Prinzip der erfindungsgemäßen Lehre zu entnehmen. In dieser ist mit 1 ein Beschleunigungsaufnehmer bzw. eine Inertialplattform bezeichnet, um die Bewegung eines 3D-Sensors bzw. Scanners 2 zu einem Objekt 3 wie Zahn bzw. Kieferbereich zu messen. Sofern sich das Objekt 3 gleichfalls bewegt, sollte auch dieses einen entsprechenden Beschleunigungsaufnehmer aufweisen bzw. diesem zugeordnet sein.

Der Scanner 2 weist einen Bildaufnahmesensor 5 auf, der mit einem Rechner 4 verbunden ist, über den die Bildausleserate des Sensors 5 geregelt bzw. gesteuert wird, wie dies zuvor erläutert worden ist. Auch umfasst der Rechner 4 eine Bildverarbeitung, um aus den von dem Sensor 5 aufgenommenen Bildern bzw. den Inhalten der einzelnen Pixel Daten zu generieren, die für die Registrierung bzw. Ermittlung des Gesamtdatensatzes benötigt werden.

Ein Mess- bzw. Datenerfassungsfeld ist mit 6 gekennzeichnet. Wird der Scanner 2 translatorisch bewegt, so werden entsprechend der Bewegungsgeschwindigkeit Bilder zeitversetzt aufgenommen, wobei im erforderlichen Umfang eine Überlappung erfolgt, um redundante Daten zu erhalten, die ein Matchen der Einzelbilder bzw. der Einzeldatensätze ermöglichen. Die versetzt zueinander verlaufenden Datenerfassungsfelder sind der Figur prinzipiell zu entnehmen. So ist ein erstes Datenfeld mit dem Bezugszeichen 6 und ein zweites Datenfeld mit dem Bezugszeichen 7 gekennzeichnet, das dann, wenn die translatorische Bewegung entsprechend dem Pfeil 8 erfolgt, vor dem Datenfeld 6 aufgenommen worden ist.

Aus der Fig. 3 ergibt sich des Weiteren, dass eine Bewegung nicht nur in Richtung des Pfeils 8, sondern in jeder Richtung des xyz-Koordinatensystems erfolgen kann, wie auch durch den Pfeil 9 angedeutet wird.

## Patentansprüche

1. Generierung eines Gesamtdatensatzes von zumindest einem Abschnitt eines Objekts (3), wie Kieferbereich, zur Ermittlung zumindest eines Charakteristikums, wie Form und Lage, durch Zusammenfügung von Einzeldatensätzen, die mittels eines relativ zu dem Objekt sich bewegenden optischen Sensors (5) als ersten Sensor und einer Bildverarbeitung ermittelt werden, wobei Einzeldatensätze von aufeinanderfolgenden Aufnahmen des Objekts redundante Daten enthalten, die zum Zusammenfügen der einzelnen Datensätze gematcht werden,
**dadurch gekennzeichnet,**
**dass** als optischer Sensor (5) eine 3D-Kamera verwendet wird,
und **dass** entweder in Abhängigkeit von der Relativbewegung zwischen dem optischen Sensor und dem Objekt (3) die Anzahl der pro Zeiteinheit ermittelten Einzeldatensätze variiert wird, wobei zur Ermittlung der Relativbewegung der erste Sensor einen zweiten Sensor (1) aus der Gruppe Beschleunigungssensor, Rotationssensor, Intertialplattform aufweist, oder in Abhängigkeit von der Anzahl der redundanten Daten von aufeinanderfolgenden Datensätzen die Anzahl der pro Zeiteinheit ermittelten Einzeldatensätze geregelt wird.

2. Generierung eines Gesamtdatensatzes nach Anspruch 1,
**dadurch gekennzeichnet,**
**dass** die Einzeldatensätze diskontinuierlich ermittelt werden.

3. Generierung eines Gesamtdatensatzes nach Anspruch 1,
**dadurch gekennzeichnet,**
**dass** die Anzahl der Einzeldatensätze pro Zeiteinheit durch Regelung und/oder Steuerung variiert wird.

4. Generierung eines Gesamtdatensatzes nach zumindest einem der vorhergehenden Ansprüche,
**dadurch gekennzeichnet,**
**dass** in Abhängigkeit von Relativgeschwindigkeit zwischen dem Objekt (3) und dem optischen Sensor (5) die Anzahl der zu ermittelnden Einzeldatensätze pro Zeiteinheit gesteuert wird.

5. Generierung eines Gesamtdatensatzes nach zumindest einem der vorhergehenden Ansprüche,
**dadurch gekennzeichnet,**
**dass** die Relativbewegung zwischen dem Objekt (3) und dem optischen Sensor (5) mittels zumindest einer Inertialplatform ermittelt wird.

6. Generierung eines Gesamtdatensatzes nach zumindest einem der vorhergehenden Ansprüche,
**dadurch gekennzeichnet,**
**dass** die Anzahl der zu ermittelnden Einzeldatensätze insbesondere bei durch Drehbewegung erfolgter Relativbewegung in Abhängigkeit vom Abstand zwischen optischem Sensor (5) und zu messendem Objekt (3) bzw. Abschnitt des Objekts verändert wird.

7. Generierung eines Gesamtdatensatzes nach zumindest einem der vorhergehenden Ansprüche,
**dadurch gekennzeichnet,**
**dass** das Objekt (3) auf einem Chip, wie CCD-Chip, des optischen Sensors (5) in Form der 3D-Kamera abgebildet wird und der Chip in Abhängigkeit von der Relativbewegung zwischen dem optischen Sensor und dem Objekt ausgelesen wird.

8. Generierung eines Gesamtdatensatzes nach Anspruch 7,
**dadurch gekennzeichnet,**
**dass** Framerate des Chips in Abhängigkeit von Relativgeschwindigkeit zwischen dem Sensor (5) und dem Objekt (3) gesteuert wird.

9. Generierung eines Gesamtdatensatzes nach Anspruch 7,
**dadurch gekennzeichnet,**
**dass** Framerate des Chips in Abhängigkeit vom Überlappungsbereich von mit dem Chip aufgenommenen und aufeinanderfolgenden Bildern geregelt wird.

10. Generierung eines Gesamtdatensatzes nach zumindest einem der vorhergehenden Ansprüche,
**dadurch gekennzeichnet,**
**dass** der optische Sensor (5) im Abstand a mit 2 mm ≤ a ≤ 20 mm zu dem Objekt (3) bewegt wird.

11. Generierung eines Gesamtdatensatzes nach zumindest einem der vorhergehenden Ansprüche,
**dadurch gekennzeichnet,**
**dass** der optische Sensor (5) derart zu dem Objekt positioniert wird, dass sich grundsätzlich ein Messfeld von 10 mm x 10 mm ergibt.

## Claims

1. A generation of a total data set of at least one section of an object (3), such as a jaw region, to determine at least one characteristic feature, such as shape and position, by combining individual data sets, which are determined by means of an optical sensor (5) as a first sensor moving relative to the object, and an image processing system, whereby individual data sets of consecutive images of the object contain redundant data, which are matched to combine the individual data sets,
**characterized in that**
a 3D camera is used as the optical sensor (5) and that either the number of individual data sets acquired per time interval are varied in dependence on the relative movement between the optical sensor and the object (3), wherein for the acquisition of the relative movement the first sensor comprises a second sensor (1) from the group acceleration sensor, rotation sensor, inertial platform, or the number of individual data sets acquired per time interval is controlled in dependence on the number of redundant data of consecutive data sets.

2. The generation of a total data set according to claim 1,
**characterized in that**
the individual data sets are acquired in a discontinuous manner.

3. The generation of a total data set according to claim 1,
**characterized in that**
the number of individual data sets per time interval is varied by closed-loop and/or open-loop control.

4. The generation of a total data set according to at least one of the preceding claims,
**characterized in that**
the number of individual data sets to be acquired per time interval is controlled in dependence on the relative movement between the object (3) and the optical sensor (5).

5. The generation of a total data set according to at least one of the preceding claims,
**characterized in that**
the relative movement between the object (3) and the optical sensor (5) is determined by means of at least one inertial platform.

6. The generation of a total data set according to at least one of the preceding claims,
**characterized in that**
the number of individual data sets to be determined is varied - in particular during relative movements resulting from rotational motion - in dependence on the distance between the optical sensor (5) and the object (3) to be measured or a section thereof.

7. The generation of a total data set according to at least one of the preceding claims,
**characterized in that**
the object (3) is imaged onto a chip, such as a CCD chip, of the optical sensor (5), such as a 3D camera, and that the chip is read out in dependence on the relative movement between the optical sensor and the object.

8. The generation of a total data set according to claim 7,
**characterized in that**
the frame rate of the chip is controlled in dependence on the relative speed between the sensor (5) and the object (3).

9. The generation of a total data set according claim 7,
**characterized in that**
the frame rate of the chip is controlled in dependence on the overlap region of consecutive images recorded by the chip.

10. The generation of a total data set according to at least one of the preceding claims,
**characterized in that**
the optical sensor (5) is moved at a distance *a* from the object (3), with 2 mm ≤ *a* ≤ 20 mm.

11. The generation of a total data set according to at least one of the preceding claims,
**characterized in that**
the optical sensor (5) is positioned relative to the object in a manner so that basically a measuring field of 10 mm x 10 mm is obtained.

## Revendications

1. Génération d'un ensemble de données d'au moins une partie d'un objet (3), par exemple une zone de mâchoire, afin de déterminer au moins une caractéristique, par exemple la forme et la position, par regroupement de données individuelles, ces dernières étant déterminées à l'aide d'un capteur optique (5) en qualité de premier capteur se déplaçant par rapport à l'objet, ainsi qu'à l'aide d'un traitement d'image, sachant que les données individuelles des prises de vues consécutives de l'objet contiennent des données redondantes servant au regroupement des données individuelles,
**caractérisée en ce**
**qu'**une caméra 3D est utilisée en tant que capteur optique (5) et que, ou bien le nombre de données individuelles à déterminer par unité de temps varie en fonction du mouvement relatif entre le capteur optique et l'objet (3), sachant que, pour déterminer le mouvement relatif, le premier capteur comprend un second capteur (1) appartenant au groupe accéléromètre, capteur de rotation, plateforme inertielle, ou bien que le nombre des données individuelles à déterminer par unité de temps est régulé en fonction du nombre de données redondantes de l'ensemble des données consécutives.

2. Génération d'un ensemble de données selon la revendication 1,
**caractérisée en ce**
**que** les données individuelles sont déterminées en discontinu.

3. Génération d'un ensemble de données selon la revendication 1,
**caractérisée en ce**
**que** le nombre de données individuelles par unité de temps varie en fonction de la régulation et/ou de la commande.

4. Génération d'un ensemble de données selon au moins l'une des précédentes revendications,
**caractérisée en ce**
**que** le nombre de données individuelles à déterminer par unité de temps est commandé en fonction de la vitesse relative entre l'objet (3) et le capteur optique (5).

5. Génération d'un ensemble de données selon au moins l'une des précédentes revendications,
**caractérisée en ce**
**que** le mouvement relatif entre l'objet (3) et le capteur optique (5) est déterminé à l'aide d'au moins une plateforme inertielle.

6. Génération d'un ensemble de données selon au moins l'une des précédentes revendications,
**caractérisée en ce**
**que** le nombre de données individuelles devant être déterminées en particulier par le mouvement relatif effectué par rotation est modifié en fonction de la distance entre le capteur optique (5) et l'objet (3) ou la partie de l'objet à mesurer.

7. Génération d'un ensemble de données selon au moins l'une des précédentes revendications,
**caractérisée en ce**
**que** l'objet (3) est reproduit sur une puce - par exemple une puce CCD - du capteur optique (5), sous forme de la caméra 3D, et que la puce est lue en fonction du mouvement relatif entre le capteur optique et l'objet.

8. Génération d'un ensemble de données selon la revendication 7,
**caractérisée en ce**
**que** le taux de rafraîchissement d'image de la puce est régulé en fonction de la vitesse relative entre le capteur (5) et l'objet (3).

9. Génération d'un ensemble de données selon la revendication 7,
**caractérisée en ce**
**que** le taux de rafraîchissement d'image de la puce est régulé en fonction de la zone de chevauchement des images consécutives enregistrées avec la puce.

10. Génération d'un ensemble de données selon au moins l'une des précédentes revendications,
**caractérisée en ce**
**que** le capteur optique (5) se déplace à une distance a de l'objet (3), sachant que : 2 mm ≤ a ≤ 20 mm.

11. Génération d'un ensemble de données selon au moins l'une des précédentes revendications,
**caractérisée en ce**
**que** le capteur optique (5) est positionné par rapport à l'objet de sorte qu'en principe il en résulte un champ de mesure de 10 mm x 10 mm.
